# EUROPEAN PATENT APPLICATION

(11) **EP 3 957 305 A1**
(43) Date of publication of application: **23.02.2022**
(21) Application number: 20805269.6
(22) Date of filing: 08.05.2020
(51) Int. Cl.: A61K 9/51, A61K 48/00, A61K 45/06, A61P 35/00

(54) **NANOPARTICLE COMPLEX FOR TREATING DISEASE, AND METHOD FOR PRODUCING SAME**

(30) Priority: 10.05.2019 KR 20190054778
(71) Applicant: Sogang University Research Foundation, Seoul 04107 (KR)
(72) Inventor: KIM, Hyun Cheol, Seoul 07344 (KR); KIM, Do Yeon, Seoul 02831 (KR); KIM, Jee Won, Goyang-si Gyeonggi-do 10371 (KR)
(74) Representative: González López-Menchero, Álvaro Luis
(86) International application number: PCT/KR2020/006109
(87) International publication number: WO 2020/231105

(57) **Abstract**

Proposed is a nanoparticle complex containing a nanoparticle that ingestible into a cell, and a lipid-based lipid structure bonded to one portion of an outer surface of the nanoparticle and improving a cellular uptake efficiency of the nanoparticle, wherein the nanoparticle contains a first reactive group, the lipid structure contains a second reactive group chemically bonded to the first reactive group of the nanoparticle, the first reactive group and the second reactive group are chemically bonded to each other, and thus the lipid structure is bonded to the nanoparticle.

<110>    SOGANG UNIVERSITY RESEARCH FOUNDATION

<120>    Nanoparticle complex with improved cellular uptake through
         surface modification using lipid and Method for manufacturing the
         same

<130>    PFAHJ-20114

<150>    KR 19/0054778
<151>    2019-05-10

<160>    2

<170>    KoPatentIn 3.0

<210>    1
<211>    21
<212>    RNA
<213>    Artificial Sequence

<220>
<223>    sense of siRNA


<400>    1
augugaaugc agaccaaaga a                                                  21


<210>    2
<211>    21
<212>    RNA
<213>    Artificial Sequence

<220>
<223>    antisense of siRNA


<400>    2
uucuuugguc ugcauucaca u                                                  21

## Description

### Technical Field

The present disclosure relates to a nanoparticle complex for disease treatment and a method of manufacturing the nanoparticle complex. More specifically, the present disclosure relates to a nanoparticle complex capable of not only causing endocytosis to occur at the cell surface, but also directly penetrating a cell membrane, with a lipid structure in the form of a tube being bonded to one portion of a surface of a nanoparticle and a method of manufacturing the nanoparticle complex. With the improved ability of the nanoparticle complex to penetrate a tissue, the nanoparticle is capable of being effectively ingested into a tumor cell in the form of a spheroid. Furthermore, a lipid structure having a sufficient length is capable of being formed on the surface of the nanoparticle by adjusting a ratio between a phospholipid to which a second reactive group that is used when a lipidome is formed is bonded and a phospholipid to which the second reactive group is not bonded.

### Background Art

The efficiency of cellular uptake is an important measure for achieving drug transport efficacy in the drug transport field. Therefore, technologies for improving the efficiency of cellular uptake have been widely developed. As an example, the technology for chemically bonding a cell-permeable peptide or the like to a nanoparticle and thus improving the efficiency of uptake of the nanoparticle is disclosed in the following patent literature.

### <Patent Literature>

Korean Patent Application Publication No. 10-2017-0040748 (published on April 13, 2017) tilted "Multi-block Polypeptides and Their Self-assembled Nanostructures for Enhanced Intracellular Delivery of Multiple Drugs")

However, the technology in the related art for improving the efficiency of cellular uptake cannot achieve a satisfactory effect. A bonded substance is easily dissolved and thus has less stability.

### Disclosure

### Technical Problem

An objective of the present disclosure, which is made in view of the problem described above is to provide a nanoparticle complex in which a surface of a nanoparticle is reformed with a lipid-based substance having high stability and excellent biocompatibility and a method of manufacturing the nanoparticle complex. The nanoparticle complex remarkably improves the efficiency of cellular uptake.

Another objective of the present disclosure is to provide a nanoparticle complex capable of not only causing endocytosis (a mechanism by which a particle having a size of 100 to 200 nm is ingested into a cell) to occur at the cell surface, but also directly penetrating a cell membrane, with a lipid structure in the form of a tube being bonded to one portion of a surface of a nanoparticle and a method of manufacturing the nanoparticle complex.

Still another objective of the present disclosure is to provide a nanoparticle complex that has the improved ability to penetrate a tissue and thus effectively ingesting a nanoparticle into a tumor cell in the form of a spheroid and a method of manufacturing the nanoparticle complex.

Still another objective of the present disclosure is to provide a nanoparticle complex capable of improving an effect of transporting a nanoparticle supporting a dielectric drug or composed of the dielectric drug itself to a cell and a tissue and thus increasing the efficiency of gene therapy and a method of manufacturing the nanoparticle complex.

Still another objective of the present disclosure is provided to a nanoparticle complex capable of being readily mass-produced through a top-down approach and a method of manufacturing the nanoparticle complex. Through the top-down approach, the lipid structure is not attached directly to the nanoparticle. That is, a lipid-based lipidome (a bubble, a liposome, or the like) and a nanoparticle are bonded to each other, the lipidome is then broken by applying a mechanical force thereto, and thus the lipid structure is formed on a surface of the nanoparticle.

Still another objective of the present disclosure is to provide a nanoparticle complex in which a lipid structure having a sufficient length is capable of being formed on a surface of a nanoparticle by adjusting a ratio between a phospholipid to which a second reactive group that is used when a lipidome is formed is bonded and a phospholipid to which the second reactive group is not bonded and a method of manufacturing the nanoparticle complex.

### Technical Solution

In order to accomplish the objectives described above, embodiments of the present disclosure are provided as follows.

According to an aspect of the present embodiment, there is provided a nanoparticle complex including: a nanoparticle that is ingested into a cell; and a plurality of lipid-based lipid structures self-assembled on one surface of the nanoparticle and improving the efficiency of cellular uptake of the nanoparticle.

In the nanoparticle complex, the nanoparticle includes a first reactive group, and some of the lipids constituting the lipid structure have a second reactive group chemically bonded to the first reactive group of the nanoparticle. Furthermore, the first reactive group and the second reactive group are preferentially chemically bonded to each other, some lipids are bonded to the nanoparticle, and this bonding leads to other lipids being self-assembled, thereby forming the lipid structure.

In the nanoparticle complex, the lipid structure may be formed by self-assembling of lipidomes, each being composed of a mixture of a lipid to which the second reactive group is bonded and a lipid to which the second reactive group is not bonded, and generation of the lipid structure bonded to the nanoparticle and formation of a shape thereof may be controlled by adjusting a mixture ratio between the lipid to which the second reactive group is bonded and the lipid to which the second reactive group is not bonded.

In the nanoparticle complex, the lipid to which the second reactive group is bonded and the lipid to which the second reactive group is not bonded may be used at a molar ratio of 1:2.33 to 99.

In the nanoparticle complex, a lipid head of the lipid structure that has hydrophilicity may be positioned on an outer lateral surface of the lipid structure, a lipid tail of the lipid structure that has hydrophilicity may be positioned inside the lipid structure, and the lipid structure may have the form of a long tube when viewed as a whole.

In the nanoparticle complex, the nanoparticle may have a diameter of 50 to 500 nm, and the lipid structure may have a length of 50 to 300 nm and may have a width of 3 to 20 nm.

The nanoparticle complex may be ingested in to the cell by endocytosis, which is a mechanism by which an existing nanoparticle enters a cell, but also directly penetrating a cell membrane and thus being ingested into the cell.

In the nanoparticle complex, the lipid structure may be capable of improving the efficiency of cellular uptake of the nanoparticle by a spheroid cell that is a cancer cell.

In the nanoparticle complex, the nanoparticle may include an anticancer agent and may be capable of improving the efficiency of death of a tumor cell having resistance to anticancer.

In the nanoparticle complex, the nanoparticle may be a nanoparticle supporting a dielectric drug or a nanoparticle composed of the dielectric drug, and may be capable of improving the efficiency of gene therapy.

According to another aspect of the present disclosure, there is provided a method of manufacturing a nanoparticle, the method including: forming a nanoparticle having a first reactive group; forming a micro-based lipid-based lipidome containing a second reactive group chemically bonded to the first reactive group; forming a lipidome-nanoparticle complex by mixing the nanoparticle and the lipidome, bonding the first reactive group and the second reactive group to each other, and thus bonding the nanoparticle to an outer surface of the lipidome; and breaking the lipidome by applying a mechanical force to the lipidome-nanoparticle complex formed and thus forming a lipid structure bonded to one portion of an outer surface of the nanoparticle, wherein in the forming of the lipidome, the lipidome is formed using a mixture of a lipid to which the second reactive group is bonded and a lipid to which the second reactive group is not bonded, the nanoparticle is ingested into a cell, and the lipid structure improves the efficiency of cellular uptake of the nanoparticle.

In the method, generation of the lipid structure bonded to the nanoparticle and formation of a shape thereof may be controlled by adjusting a mixture ratio between the lipid to which the second reactive group is bonded and the lipid to which the second reactive group is not bonded.

In the method, in the breaking of the lipidome, in a case where a mechanical force is applied to the lipidome-nanoparticle complex and after a predetermined time elapses, the lipidome is broken, phospholipids that constitute the lipidome are recombined, and thus the lipid structure in the form of a tube that is bonded to the nanoparticle is formed.

### Advantageous Effects

The embodiments of the present disclosure can provide the following effects.

According to the embodiments of the present disclosure, a surface of a nanoparticle is reformed with a lipid-based substance having high stability and excellent biocompatibility. Thus, the effect of remarkably improving the efficiency of cellular uptake can be achieved.

In addition, according to the embodiments of the present disclosure, a lipid structure in the form of a tube is formed on one portion of a surface of a nanoparticle. Thus, the effect of not only causing endocytosis (a mechanism by which a particle having a size of 100 to 200 nm is ingested into a cell) to occur at the cell surface, but also directly penetrating a cell membrane can be achieved.

In addition, according to the embodiments of the present disclosure, with the improved ability to penetrate a tissue, the effect of ingesting the nanoparticle into a tumor cell in the form of a spheroid can be achieved.

In addition, according to the embodiments of the present disclosure, the effect of readily mass-producing a nanoparticle complex through a top-down approach can be achieved. Through the top-down approach, a lipid structure is not attached directly to the nanoparticle. That is, a lipid-based lipidome (a bubble, a liposome, or the like) and a nanoparticle are bonded to each other, the lipidome is then broken by applying a mechanical force thereto, and thus the lipid structure is formed on the surface of the nanoparticle.

In addition, according to the embodiments of the present disclosure, the effect of forming a lipid structure having a sufficient length can be achieved. This lipid structure can be formed by adjusting a ratio between a phospholipid to which a second reactive group that is used when a lipidome is formed is bonded and a phospholipid to which the second reactive group is not bonded.

### Description of Drawings

FIG. 1 is a schematic view illustrating a nanoparticle complex according to a first embodiment of the present disclosure;
FIG. 2 is a TEM image illustrating the nanoparticle complex according to the first embodiment of the present disclosure;
FIG. 3 is a Cry-TEM image illustrating the nanoparticle complex according to the first embodiment of the present disclosure;
FIG. 4 is a Cry-TEM image illustrating a lipid structure that is bonded to the nanoparticle complex according to the first embodiment of the present disclosure;
FIG. 5 is a Cry-TEM image illustrating the nanoparticle complexes according to the first embodiment of the present disclosure that are manufactured at different ratios between phospholipids;
FIG. 6 is a TEM image illustrating a virus nanoparticle that is used for a nanoparticle complex according to a second embodiment of the present disclosure;
FIG. 7 is a Cryo-TEM image illustrating the nanoparticle complex according to the second embodiment of the present disclosure;
FIG. 8 is a graph illustrating the results of analysis that is performed using a flow cytometry to verify the efficiency of cellular uptake of the nanoparticle complex according to the first embodiment of the present disclosure;
FIGS. 9 and 10 are images each obtained by a confocal microscope to verify the efficiency of cellular uptake of the nanoparticle complex according to the first embodiment of the present disclosure;
FIG. 11 is an image obtained by the confocal microscope to verify the efficiency of cellular uptake of the nanoparticle complex according to the second embodiment of the present disclosure;
FIG. 12 is an image obtained by the confocal microscope to verify the efficiency of cellular uptake of a nanoparticle complex according to a third embodiment of the present disclosure;
FIGS. 13 and 14 are graphs each illustrating the results of analysis that is performed using the flow cytometry to verify the efficiency of cellular uptake of each of the nanoparticle complexes that are manufactured using various lipids and reactive groups;
FIG. 15 is a graph illustrating the results of analysis that is performed using the flow cytometry to verify the efficiency of cellular uptake of the nanoparticle complex according to the first embodiment of the present disclosure after treatment with an endocytosis inhibitor;
FIG. 16 is a graph illustrating the results of analysis that is performed using the flow cytometry to verify the efficiency of cellular uptake of each of the nanoparticle complexes according to the first embodiment of the present disclosure that are manufactured at different ratios between the phospholipids after the treatment with the endocytosis inhibitor;
FIG. 17 is an image obtained by the confocal microscope to verify the efficiency of cellular uptake of the nanoparticle complex according to the third embodiment of the present disclosure after the nanoparticle complex is excessively treated with HA;
FIG. 18 is a graph illustrating the results of cell viability assay to verify the efficacy of the nanoparticle complex, as an anticancer agent transporter, according to the first embodiment of the present disclosure;
FIGS. 19 and 20 are a view and a graph, respectively, illustrating the results of gene silencing to verify the efficacy of the nanoparticle complex, as a dielectric drug transporter, according to the third embodiment of the present disclosure;
FIGS. 21 and 22 are a view and a graph, respectively, illustrating the results of gene silencing to verify the efficacy of the nanoparticle complex, as the dielectric drug transporter, according to the third embodiment of the present disclosure;
FIG. 23 is an image obtained by the confocal microscope to verify the efficiency of cellular uptake of the nanoparticle complex according to the first embodiment in a spheroid tumor cell model;
FIG. 24 is an image obtained by the confocal microscope to verify the efficiency of cellular uptake of the nanoparticle complex according to the third embodiment of the present disclosure in the spheroid tumor cell model;
FIGS. 25 and 26 are a view and a graph, respectively, illustrating the results of gene silencing to verify the efficacy of the nanoparticle complex, as the dielectric drug transporter, according to the third embodiment of the present disclosure in the spheroid tumor cell model; and
FIG. 27 is an image obtained by the confocal microscope to verify the efficiency of cellular uptake of the nanoparticle complex according to the third embodiment of the present disclosure in an animal model.

### Description of the Reference Numerals in the Drawings

- 1:: nanoparticle
- 2:: lipid structure
- 3:: lipid head
- 4:: lipid tail

### Mode for Invention

A nanoparticle complex for disease treatment and a method of preparing the nanoparticle complex for disease treatment according to the present disclosure will be described in detail below with reference to the accompanying drawings. Unless particularly defined otherwise, meanings of all terms used throughout the present specification are the same as those thereof that are normally understood by a person of ordinary skill in the art to which the present disclosure pertains. If an interpretation conflict as to the meaning of the term used throughout the present specification occurs, the definition of the term in the present specification governs. In addition, detailed descriptions of a known function and configuration that would unnecessarily make the nature and gist of the present disclosure indefinite are omitted. Unless particularly described otherwise, throughput the present specification, the expression "includes a constituent element" means "further may include any other constituent element, not "excludes any other constituent element".

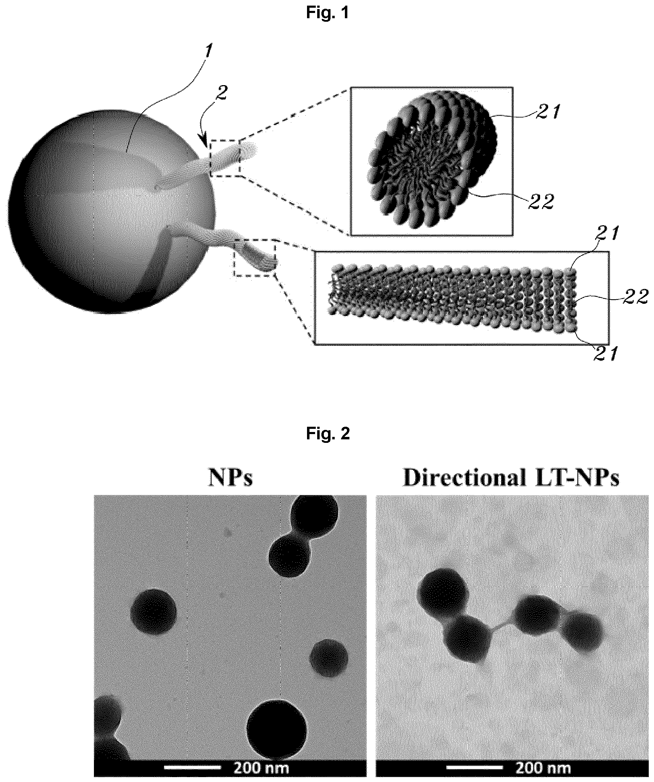

With reference to FIGS. 1 to 24, a nanoparticle complex for disease treatment according to an embodiment of the present disclosure includes a nanoparticle 1, a lipid-based lipid structure 2, and the like. The nanoparticle 1 is ingested into a cell. The lipid-based lipid structure 2 is bonded to one portion of an outer surface of the nanoparticle 1 and thus is capable of improving the efficiency of cellular uptake of the nanoparticle 1.

The nanoparticle 1 is configured in a manner that is ingested into the cell and may be used for disease treatment and the like. The nanoparticle 1 is composed of, for example, a substance capable of supporting a drug or treating a disease. There are various nanoparticles in the related art that may be ingested into the cell and thus may be used for disease treatment. Examples of the nanoparticle may include an albumin nanoparticle, an nanoparticle composed of hexane and albumin, a biodegradable polymeric nanoparticle, a hexane nanoparticle, a virus particle, and the like that are used for disease treatment. The nanoparticle 1 described above may have a diameter of, for example, 50 to 500 nm, a spherical form, and the like. The nanoparticle 1 may include a chemical reactive group (hereinafter referred to as a "first reactive group) that is bonded to a lipid structure. For example, the first reactive group may be a compound including a thiol group, an amine group, an amino group, a carboxy group, and the like.

The lipid structure 2 is a lipid-based structure that is bonded to one portion of the outer surface of the nanoparticle 1 and thus improves the efficiency of cellular uptake of the nanoparticle 1. The lipid structure 2 may have, for example, a length of 50 to 300 nm and a width of 3 to 20 nm. One or more lipid structures 2 may be bonded to the outer surface of the nanoparticle 1. In addition, the lipid structure 2 may include a chemical reactive group (hereinafter referred to as a "second reactive group") that is chemically bonded to the first reactive group of the nanoparticle 1. For example, the second reactive group may be a compound including a thiol group, an amine group, an amino group, a carboxy group, and the like. The lipid structure 2 is formed by recombining lipidomes each of which is composed of a mixture of a lipid to which the second reactive group is bonded and a lipid to which the second reactive group is not bonded. Generation of the lipid structure 2 and formation of a shape (a pattern, a length, and the like) thereof may be controlled by adjusting a mixture ratio between the lipid to which the second reactive group is bonded and the lipid to which the second reactive group is not bonded. It is desirable that the lipid to which the second reactive group is bonded is mixed and the lipid to which the second reactive group is not bonded are mixed at a molar ratio of 1:2.33 to 99.

For example, a lipid head 21 having hydrophilicity is positioned on an outer lateral surface of the lipid structure 2, and a lipid tail 22 having hydrophobicity is positioned inside the lipid structure 2. The lipid structure 2 may have the form of a long tube when viewed as a whole. The second reactive group positioned on one end portion of the lipid structure 2 in the form of a long tube is chemically bonded to the first reactive group of the nanoparticle 1. Thus, the lipid structure 2 in the form of a long tube is bonded to the outer surface of the nanoparticle 1. For example, in a case where the nanoparticle 1 is an albumin nanoparticle and where an N-hydroxysuccinimide (NHS) reactive group is formed on the lipid structure 2, the nanoparticle 1 and the lipid structure 2 may be bonded to each other through a NHS-amine reaction. According to the present disclosure, a surface of the nanoparticle 1 is reformed with a lipid-based substance having high stability and excellent biocompatibility, and thus the efficiency of cellular uptake of the nanoparticle 1 may be remarkably improved. A mechanism by which the nanoparticle having a size of 100 to 200 nm is ingested in the cell is endocytosis. The nanoparticle complex in which the lipid structure 2 in the shape of a tube is bonded to one portion of the surface of the nanoparticle 1 may not only be ingested in the cell by endocytosis, but may also directly penetrate a cell membrane. For this reason, the nanoparticle may be effectively ingested into a tumor cell in the form of a spheroid and thus may also find application in a living body model.

A method of preparing a nanoparticle complex according to a second embodiment of the present disclosure includes a nanoparticle formation step of forming a nanoparticle having a first reactive group, a lipidome formation step of forming a micro-sized lipid-based lipidome (a bubble, a liposome, or the like) that includes a second reactive group chemically bonded to the first reactive group; a lipid complex formation step of forming a lipidome-nanoparticle complex by mixing the nanoparticle and the lipidome, bonding the first reactive group and the second reactive group to each other, and thus bonding the nanoparticle to an outer surface of the lipidome, a breakage step of breaking the lipidome by applying a mechanical force to the lipidome-nanoparticle complex formed in the lipid complex formation step and thus of forming a lipid structure bonded to one portion of an outer surface of the nanoparticle, and the like.

In the nanoparticle formation step, the nanoparticle that is ingested into the cell and is used for disease treatment is formed in such a manner as to include the first reactive group. Various methods in the related art may be used to manufacture the nanoparticle. For example, in the case of the albumin nanoparticle supporting a drug, an amine group is included in albumin, and may be used as the first reactive group. In the case of a siRNA nanoparticle suppressing expression of particular protein, the siRNA nanoparticle is coated with hyaluronic acid to which amine is attached, and thus the first reactive group may be formed on the siRNA nanoparticle.

In the lipid complex formation step, the micro-sized lipid-based lipidome (the bubble, the liposome, or the like) that includes the second reactive group which is chemically bonded to the first reactive group is formed. For example, the bubble is formed of a lipid (for example, a phospholipid) and is filled with gas. The second reactive group is positioned in the bubble. The micro-sized bubble in the form of a liposome that has gas inside and is composed of lipids may be manufactured by a manufacturing method in the related art. For example, the lipid to which the second reactive group is bonded and the lipid to which the second reactive group is not bonded are mixed at a predetermined ratio in the presence of an organic solvent, and thus, a lipid film is formed. Then, the lipid film is dissolved in a solvent, and gas is injected. In this manner, the bubble may be formed. The generation of the lipid structure 2 and the formation of a shape thereof (a pattern, a length, and the like) may be controlled by adjusting the mixture ratio between the lipid to which the second reactive group is bonded and the lipid to which the second reactive group is not bonded. It is desirable that the lipid to which the second reactive group is bonded and the lipid to which the second reactive group is not bonded are mixed at a molar ratio of 1:2.33 to 99.

In the lipid complex formation step, the nanoparticle and the lipidome are mixed, the first reactive group and the second reactive group are bonded to each other, and the nanoparticle is bonded to the outer surface of the lipidome. Thus, a lipidome-nanoparticle complex is formed.

In the breakage step, the lipidome is broken by applying a mechanical force to the lipidome-nanoparticle complex formed in the lipid complex formation step. Thus, the lipid structure bonded to one portion of the outer surface of the nanoparticle is formed. In a case where a mechanical force is applied to the lipidome-nanoparticle complex using an ultrasonic device or the like and after a predetermined time elapses, the lipidome is broken, lipids that constitute the lipidome are recombined, and thus the lipid structure in the form of a tube that is bonded to the nanoparticle is formed. Specifically, when a mechanical force is applied to the lipidome-nanoparticle complex, the lipidome is broken. Then, some lipids having the second reactive group are preferentially bonded to the nanoparticles (the first reactive group and the second reactive group are chemically bonded to each other), and this bonding leads to the other lipids being self-assembled. Thus, the lipid structure is formed.

According to the present disclosure, the nanoparticle complex is capable of being readily mass-produced through a top-down approach. Through the top-down approach, the lipid structure is not attached directly to the nanoparticle. That is, the lipid-based lipidome and the nanoparticle are bonded to each other, the lipidome is then broken by applying a mechanical force thereto, and thus the lipid structure is formed on the surface of the nanoparticle.

Embodiments of the present disclosure will be described in more detail below. However, the scope of the present disclosure is not limited to the embodiments.

### <Implementation Example 1> Manufacturing of an Albumin Nanoparticle Complex

### 1. Preparation of Albumin Nanoparticles (NPs)

Albumin (human serum albumin) was dissolved at a concentration of 20 mg/mL in distilled water, a pH of the resulting solution was then adjusted to a pH of 8 by addition of 0.2 M NaOH, and thus an albumin solution was prepared. 100% ethanol titration was performed at a flow rate of 1 mL/min in the albumin solution. Subsequently, 10µL of 4% glutaraldehyde was added, and ethanol was left to evaporate in the absence of light for one night. Centrifugation was formed at 13200 rpm for 10 minutes, and ungranulated albumin was removed using a pipette. Re-dispersion was performed with PBS and centrifugation was formed at 3000 rpm for 5 minutes. Then, supernatant liquid (nanoparticles (NPs)) was obtained except for a micropellet using the pipette (when a fluorescence experiment was conducted, fluorescent dye (if necessary) and nanoparticles were left to react at room temperature for one night, centrifugation was performed at 13200 rpm for 10 minutes, the fluorescent dye that did not react was removed using the pipette, and then, for use, re-dispersion was performed with PBS.

### 2. Preparation of a Lipidome (Liposome)

Lipids, DSPC (1,2-distearoyl-sn-glycero-3-phosphocholine) and DSPE-PEG-NHS2000 (1,2-distearoyl-sn-glycero-3-phosphoethanolamine-N-polyethyle ne glycol succinimidyl ester) were mixed at a molar ratio of 9.25:0.75 and were dissolved at a concentration of 10 mg/ml. This mixture of these lipids was dissolved at a concentration of 10 mg/ml in chloroform, and 100 ul was put into each Ic vial 1 in such a manner that 1 mg/ml was obtained. Then, the chloroform was left to evaporate using nitrogen gas. Then, drying was performed under vacuum for one or more hours using an desiccator. Thus, a lipid film was formed. 1 ml of auto PBS was put into the lipid film vial, and thus a lipid solution was formed. A HPLC vial containing the lipid solution was immersed in water having a temperature of 55C° or higher until the lipid solution had a temperature of 55C° or higher. Sonication was performed for 15 seconds in a bath sonicator (immersion in hot water and sonication were repeated approximately 3 times), and thus a liposome was formed.

### 3. Preparation of the Lipidome (the Bubble that Results from Using the Lipid)

C₃F₈ gas was injected for 30 seconds into the vial containing the liposome obtained by performing the second process in Implementation Example 1, and the liposome and C₃F₈ gas was mixed for 45 seconds using a vial mixer. Thus, a lipid-based microbubble containing a NHS reactive group was formed.

### 4. Formation of a Nanoparticle Complex (Directional LT-NPs)

(1) The nanoparticle formed in the first process in Implementation Example 1 was immersed in a liposome solution (formed by mixing the liposome formed in the second process in Implementation Example 1 with PBS at a concentration of 1 mg/ml), and then was left to react at RT for two or more hours in order to induce an NHS-amine reaction (at this time, a liposome-nanoparticle complex (liposome-NPs) was formed). Then, a mechanical force was applied to the liposome-nanoparticle complex for five or more minutes under the condition that power = 2 W, frequency = 1 MHZ, and duty cycle = 100%, using the ultrasonic device. Subsequently, the broken liposome was incubated at RT for one or more hours in such a manner that the lipid structure was sufficiently formed. Thus, a nanoparticle complex (directional LT-NPs) was formed as a result of the lipid structure being bonded to one portion of the outer surface of the nanoparticle.
(2) Other conditions were set up to be the same as those in (1) of the fourth process in Implementation Example 1, except that the microbubble formed in the third process in Implementation Example 1 was used instead of the liposome. Then, a nanoparticle complex (directional LT-NPs) was formed as a result of the lipid structure being bonded to one portion of the outer surface of the nanoparticle.
(3) Other conditions were set up to be the same as those in (2) of the fourth process in Implementation Example 1, except that a mixture ratio between DSPC and DSPE-PEG-NHS2000 was set differently or except that DSPE-PEG was used instead of DSPE-PEG-NHS2000. Then, a nanoparticle complex (directional LT-NPs) was formed as a result of the lipid structure being bonded to one portion of the outer surface of the nanoparticle. Nanoparticle complexes that were manufactured at mixture ratios of 9.75:0.25, 9.5:0.5, 9:1, 8:2, and 7:3 between DSPC and DSPE-PEG-NHS2000 using DSPE-PEG instead of DSPE-PEG-NHS2000 are represented by the term NHS X in the following figures.

### <Implementation Example 2> Manufacturing of a Virus Nanoparticle Complex

### 1. Preparation of a Virus Nanoparticle

An AAV2-GFP (vector Biolabs, 1x1013GC/ml, 20ul) particle (AAV) was used as a virus nanoparticle.

### 2. Preparation of the Lipidome (the Bubble that Results from Using the Lipid)

Lipids, DSPC (1,2-distearoyl-sn-glycero-3-phosphocholine) and DSPE-PEG2k (1,2-distearoyl-sn-glycero-3-phosphoethanolamine-N-[succinyl (polyethylene glycol)-2000]) were mixed at a molar ratio of 9.5:0.5, and then a mixture of these lipids was dissolved in chloroform in such a manner that 1 mg/vial was obtained. The chloroform was evaporated using nitrogen gas. Subsequently, PBS was added in such a manner that a concentration of dried phospholipid is 1 mg/ml, and immersion in D.W having a temperature of 55C° or higher took place for five seconds. Then, the phospholipid dried for 30 seconds was dispersed into aqueous phase using the bath sonicator. This process was repeated approximately three times, and thus the dried phospholipid was completely dispersed into aqueous phase. Then, a vial was fully filled with C₃F₈ gas, and was shaken for 45 seconds. Thus, the lipid-based microbubble was formed.

### 3. Formation of a Nanoparticle Complex (Directional LT-AAV)

The virus nanoparticles prepared in the first process in Implementation Example 2 and the microbubble prepared in the second process in Implementation Example 2 were mixed and were caused to react at room temperature for three or more hours. Then, a mechanical force was applied to the mixture for 30 seconds using an ultrasonic wave (2.0 W/cm²). Subsequently, the broken microbubble was caused to react at room temperature for 1 or more hours in such a manner that the lipid structure was sufficiently formed. Thus, a nanoparticle complex (directional LT-AAV) was formed as a result of the lipid structure being bonded to one portion of the outer surface of the nanoparticle.

### <Implementation Example 3> Manufacturing of a Nanoparticle Complex Composed of Albumin and RNA

### 1. Preparation of nanoparticles (siRNA NPs) composed of Albumin and RNA

Albumin (human serum albumin) was dissolved at a concentration of 40 mg/mL in 0.1 mM HEPES with 0.01 mM EDTA, and then thiol-modify VEGF siRNA duplex (5' modified) was added. 100% ethanol titration was performed at a flow rate of 1 mL/min until a mixed solution of albumin and siRNA became turbid. Then, the ethanol was left to evaporate for one night in the absence of light. Centrifugation was performed at 13200 rpm for 10 minutes, and then, an ungraduated substance was removed using the pipette. Re-dispersion was performed with PBS. Centrifugation was performed at 3000 rpm for 5 minutes. Then, a supernatant (composed of siRNA and albumin) was obtained except for a micropellet using the pipette. A sense strand ofthiol-modify VEGF siRNA duplex is 5'-AUGUGAAUGCAGACCAAAGAATT-3'(Sequence ID: 1), and an antisense strand thereof is 5'-thiol-UUCUUUGGUCUGCAUUCACAUTT-3'(Sequence ID: 2).

### 2. Formation of a Nanoparticle Complex (Directional siRNA LT-NPs)

The nanoparticle composed of RNA and albumin that was formed in the first process in Implementation Example 3 were immersed in a bubble solution (formed as a result of mixing the bubble formed in the third process in Implementation Example 1 with PBS at a concentration of 1 mg/ml), and then was caused to react at RT for 2 or more hours. Then, a mechanical force was applied for five or more minutes under the condition that power = 2 W, frequency = 1 MHZ, and duty cycle = 100%, using the ultrasonic device. Subsequently, the broken bubble was incubated at RT for 1 or more hours in such a manner that the lipid structure was sufficiently formed. Thus, a nanoparticle complex (directional siRNA LT-NPs) was formed as a result of the lipid structure being bonded to one portion of the outer surface of the nanoparticle.

### <Implementation Example 4> Manufacturing of an RNA Nanoparticle Complex that is Coated Sequentially with Cationic Polymer and Hyaluronic Acid

### 1. Preparation of RNS nanoparticles (siRNA NPs) Coated Sequentially with Cationic Polymer and Hyaluronic Acid

A sense strand of vascular endothelial growth factor A (VEGF-A)) in a rat is 5'-AUGUGAAUGCAGACCAAAGAATT-3' (Sequence ID:1), and an antisense strand thereof is 5'-UUCUUUGGUCUGCAUUCA CAUTT-3' (Sequence ID:2) Long linear single-stranded DNA that results from encoding the sense and the antisense was manufactured. Along with T7 RNA polymerase, a circular DNA template that became a ligase was cultivated at a temperature of 37C° for 20 hours in a reaction buffer (8 mM Tris-HCI, 0.4 mM spermidine, 1.2 mM MgCl₂, and 2 mM dithiothreitol). The generated solution was pipetted several times, was ultrasonicated for 5 minutes, and thus was broken down into particles. The solution was centrifuged at 13,200 rpm for 20 minutes, and a supernatant was removed. Thereafter, RNase-free water was added, and the particles were washed. The solution was re-ultrasonicated for one minute and was centrifuged. A cleaning step was further repeated three times, and thus RCT reagent was removed. Thus, RNA-microsponge (anti-VEGF siRNA hydrogel) was obtained. The siRNA hydrogel was put in the bath sonicator for one or more minutes, and was well stirred in such a manner as not to be lumpy. Then, centrifugation was performed for several seconds using a mini-centrifuge to the degree to which a drop was splashed on a wall and fell down. Then, 3µl of siRNA hydrogel and 12µl of nuclease free water were mixed, and thus a first solution was formed. Furthermore, 1µl of 1 mg/ml bPEI and 9µl of nuclease free water were mixed, and thus a second solution was formed. Then, the first solution and the second solution were caused to react at room temperature for 10 minutes, and 5µl of 1mg/ml hyaluronic acid-amine was slowly added. Then, the first solution and the second solution were caused to react at room temperature for 10 minutes. Then, lumpy particles were stirred in such a manner as not to be lumpy, using the sonicator. Thus, RNA nanoparticles (siRNA NPs) coated sequentially with cationic polymer and hyaluronic acid was obtained. Manufacturing of the RNA nanoparticle coated sequentially with cationic polymer and hyaluronic acid is described above with reference to Korean Patent No.10-1880790 invented by the inventor of the present application.

### 2. Formation of a Nanoparticle Complex (lipid-siRNA NPs(siRNA LT-NPs))

The RNA nanoparticle coated sequentially with cationic polymer and hyaluronic acid that was formed in the first process in Implementation Example 4 was immersed in the bubble solution (formed as a result of mixing the bubble formed in the third process in Implementation Example 1 with PBS at a concentration of 1 mg/ml), and then was caused to react at RT for two or more hours. Then, a mechanical force was applied for five or more minutes under the condition that power = 2 W, frequency = 1 MHZ, and duty cycle = 100%, using the ultrasonic device. Subsequently, the broken bubble was incubated at RT for one or more hours in such a manner that the lipid structure was sufficiently formed. Thus, a nanoparticle complex (lipid-siRNA NPs(siRNA LT-NPs)) was formed as a result of the lipid structure being bonded to one portion of the outer surface of the nanoparticle that was coated sequentially with cationic polymer and hyaluronic acid.

### <Implementation Example 5> Manufacturing of a Nanoparticle Complex Using Various Types of Lipids and Reactive Groups

1. Other conditions were set up to be the same as those in (2) of the fourth process in Implementation Example 1, except that DPPC (1,2-dipalmitoyl-sn-glycero-3-phosphocholine), DOPE (1,2-dioleoyl-sn-glycero-3-phosphoethanolamine), and cis-PC (1,2-dioleoyl-sn-glycero-3-phosphocholine) were used instead of DSPC and that the lipid (DSPC, DPPC, DOPE, or cis-PC) to which the second reactive group was not bonded and the lipid (DSPE-PEG-NHS2000) to which the second reactive group was bonded were mixed at a molar ratio of 9.5:0.5. Thus, a nanoparticle complex was formed as a result of the lipid structure being bonded to one portion of the outer surface of the nanoparticle.
2. Other conditions were set up to be the same as those in (2) of the fourth process in Implementation Example 1, except that DSPE-PEG2000-SH and DSPE-PEG2000-Amine were used instead of DSPE-PEG-NHS2000 and that the lipid (DSPC) to which the second reactive group was not bonded and the lipid (DSPE-PEG-NHS2000, DSPE-PEG2000-SH, or DSPE-PEG2000-Amine) to which the lipid was bonded were mixed at a molar ratio of 9.6:0.5. Thus, a nanoparticle complex was formed as a result of the lipid structure being bonded to one portion of the outer surface of the nanoparticle.

### <Implementation Example 6> Verification of a Property of the Nanoparticle Complex

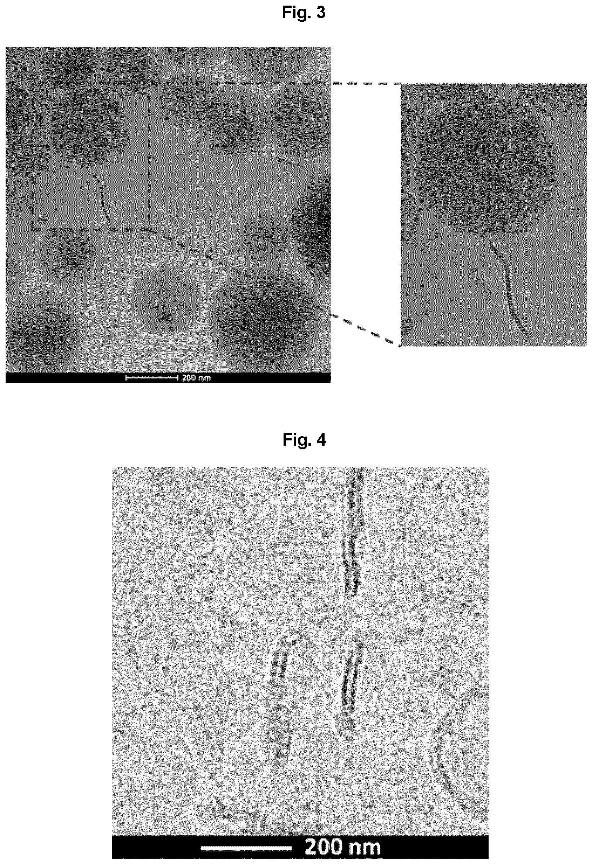

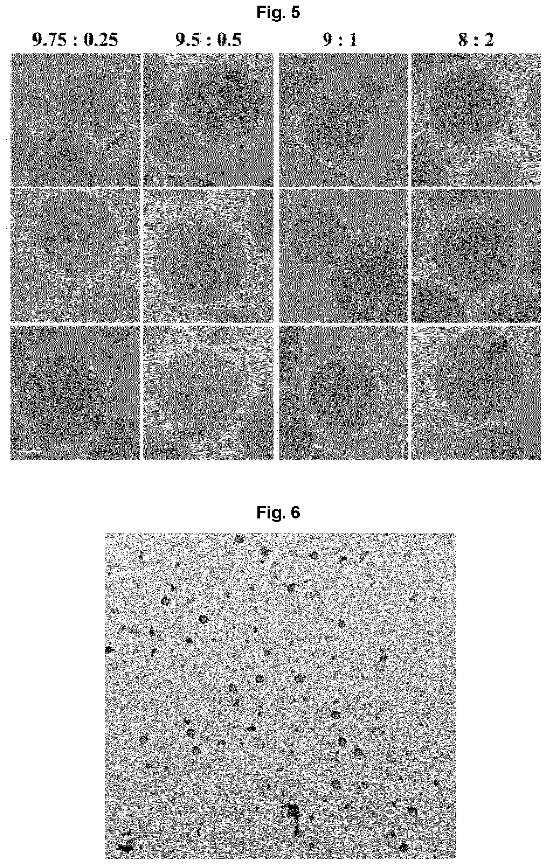

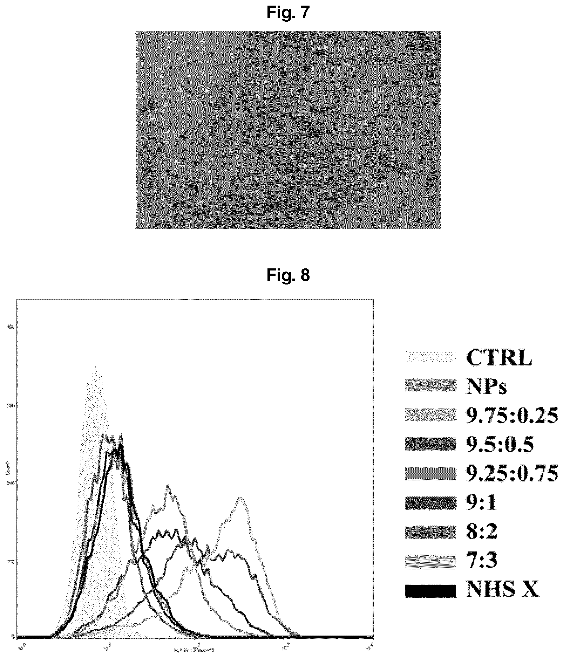

1. The nanoparticles (NPs) formed in the first process in Implementation Example 1 and the nanoparticles (directional LT-NPs) formed in (2) of the fourth process in Implementation Example 1 were measured with a TEM. The results of the measurements are shown in FIG. 2. The nanoparticle complex and the lipid structure formed in (2) of the fourth process in Implementation Example 1 were measured with a Cryo-TEM. The results of the measurements are shown in FIGS. 3 and 4, respectively. The nanoparticle complexes formed in (3) of the fourth process in Implementation Example 1 were measured with the Cryo-TEM. The results of the measurement are shown in FIG. 5 (only the nanoparticle complexes of which the mixture ratios between DSPC and DSPE-PEG-NHS2000 were 9.75:0.25, 9.5:0.5, 9:1, and 8:2, respectively, were measured). The virus nanoparticle prepared in the first process in Implementation Example 2 was measured with the TEM. The results of the measurement are shown in FIG. 6. The nanoparticle complex (directional LT-AAV) formed in the third process in Implementation Example 2 was measured with the Cryo-TEM. The results of the measurement are shown in FIG. 7.
2. From FIG. 2, it can be seen that a surface of the albumin nanoparticle is smooth and that in contrast, the surface of the nanoparticle in the albumin nanoparticle complex to which the lipid structure is attached is not smooth. In addition, from FIG. 3, it can be seen that the lipid structure in the form of a tube is attached to the surface of the nanoparticle. From FIG. 4, it can be seen more clearly that the lipid structure has the form of a tube. In addition, from FIGS. 3 and 4, it can be seen that the lipid structure has a length of 50 to 300 nm and has a width of 3 to 20 nm. Furthermore, it can be seen that one or more lipid structures are possibly bonded to the outer surface of the nanoparticle. In addition, from FIGS. 3 and 5, it can be seen that the lipid structure varies in pattern according to the ratio of between DSPC and DSPE-PEG-NHS. Furthermore, it can be seen that the more increased an amount of DSPE-PEG-NHS, the more decreased a length of the lipid structure and the less formed the lipid structure on the nanoparticle. In addition, from FIG. 6, it can be seen that the surface of the virus nanoparticle is smooth. In contract, from FIG. 7, it can be seen that the lipid structure in the form of a tube is attached to the surface of the virus nanoparticle.

### <Implementation Example 7> Evaluation of Efficiency of Cellular Uptake of the Nanoparticle Complex

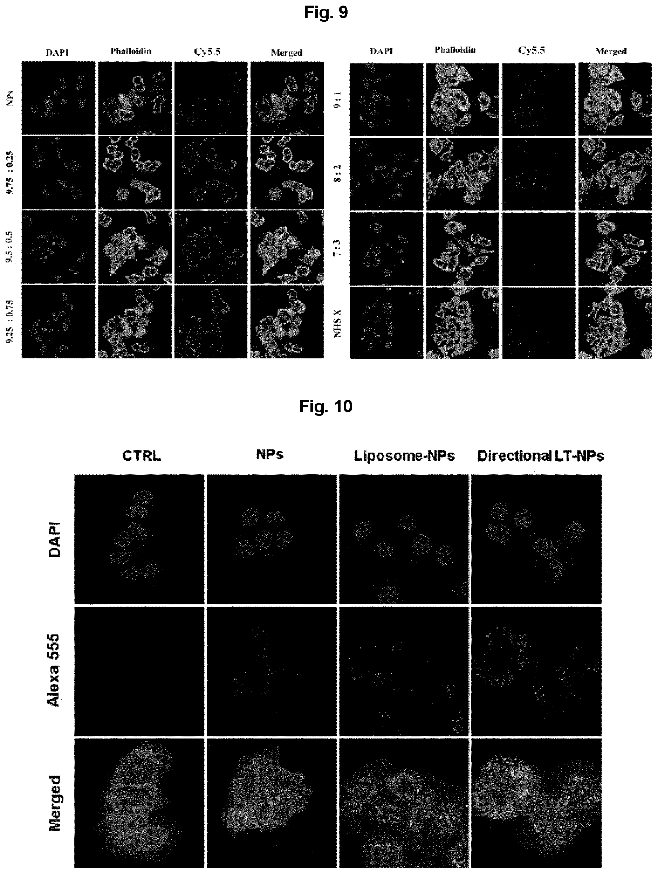

1. Using a flow cytometry, analysis was performed in order to evaluate the efficiency of cellular uptake of each of the nanoparticles (NPs) formed in the first process in Implementation Example 1 and the nanoparticle complex (directional LT-NPs) formed in (2) and (3) of the fourth process in Implementation Example 1. The results of the analysis are shown in FIG. 8. The results of quantification are shown in Table 1. Imaging was performed using a confocal microscope, and the results of the imaging are shown in FIG. 9. A549 cells (1 × 10⁴) were treated with the nanoparticle and the nanoparticle complex that were marked with Alexa 488 fluorescent dye, and then analysis was performed using the flow cytometer. The A549 cells (1 × 10⁵) of which nuclei were stained using DAPI, and of which cytoskeletons thereof were stained using phalloidin were treated with the nanoparticle and the nanoparticle complex that were marked with Cy5.5 fluorescent dye, and then analysis was performed using the confocal microscope.

**Table 1**

| | CTRL | NPs | 9.75:0.25 | 9.5:0.5 | 9.25:0.75 | 9:1 | 8:2 | 7:3 | NHSX |
|---|---|---|---|---|---|---|---|---|---|
| Alexa 488+ | 0.43 | 18.30 | 95.91 | 92.51 | 77.23 | 78.17 | 20.90 | 10.97 | 22.27 |
| STD | 0.19 | 0.24 | 0.24 | 0.11 | 0.26 | 0.37 | 0.36 | 0.05 | 0.17 |

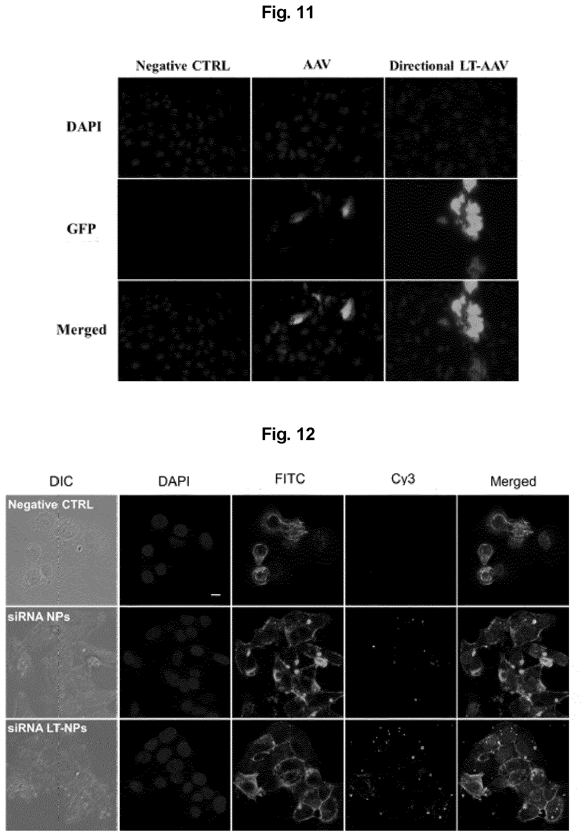

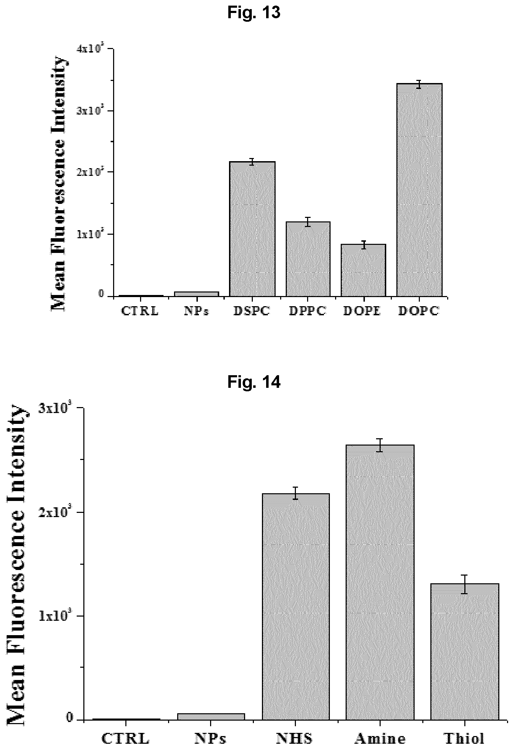

2. Using the confocal microscope, imaging was performed in order to evaluate the efficiency of cellular uptake of each of the nanoparticles (NPs) formed in the first process in Implementation Example 1 and liposome-nanoparticle complex (liposome-NPs) and nanoparticle complex (directional LT-NPs) that were formed in (1) of the fourth process in Implementation Example 1. The results of the imaging are shown in FIG. 10. A549 cells (1 × 10⁵) of which nuclei were stained using DAPI were treated with the nanoparticle, the liposome-nanoparticle complex, and the nanoparticle complex that were marked with Alexa 555 fluorescent dye. Then, analysis was performed using the confocal microscope.
3. In addition, imaging was performed in order to evaluate the efficiency of cellular uptake of each of the nanoparticle (AAV) prepared in the first process in Implementation Example 2 and the nanoparticle complex (directional LT-AAV) formed in the third process in Implementation Example 2. The results of the imaging are shown in FIG. 11. APRE19 cells (1 × 10⁵) of which nuclei were stained using DAPI were treated with the virus nanoparticle and the nanoparticle complex. Analysis was performed using the confocal microscope.
4. In addition, using the confocal microscope, imaging was performed in order to evaluate the efficiency of cellular uptake of each of the nanoparticles (siRNA NPs) prepared in the first process in Implementation Example 4 and the nanoparticle complex (siRNA LT-NPs) formed in the second process according to Implementation Example 4. The results of the imaging are shown in FIG. 12. MCF-7 cells (1 × 10⁵) of which nuclei were stained using DAPI and of which cytoskeletons were stained using FITC were treated with the nanoparticle and the nanoparticle complex that were marked with Cy3 fluorescent dye. Then, analysis was performed using the confocal microscope.
5. In addition, A549 cells (1 × 10⁵) were treated with the nanoparticle complexes formed in the first and second processes in Implementation Example 5, which were marked with Alexa 647 fluorescent dye. Then fluorescence intensity was measured. The results of the measurement are shown in FIGS. 13 and 14. DSPC in FIG. 13 indicates the results of evaluating the result of the nanoparticle complex that was manufactured using DSPC. Thiol in FIG. 14 indicates the results of the nanoparticle complex that was manufactured using DSPE-PEG2000-SH.
6. From FIG. 8 and Table 1, it can be seen that the nanoparticle complex has much higher cellular uptake efficacy than the nanoparticle. It can be seen that a ratio of an Alexa 488 positive cell (cell into which the dyed nanoparticle was ingested) is 78% or greater than in a control example when the ratio of DSPC to DSPE-PEG-NHS2000 is equal to or higher than a ratio of 9:1. It can be seen that in a case where the amount of DSPE-PEG-NHS2000 is further increased, the efficiency of cellular uptake falls remarkably. Thus, it can be understood that in a case where the lipid structure having a sufficient length and shape are not formed on the surface of the nanoparticle, the efficiency of cellular uptake efficiency falls. In addition, from FIG. 9, it can be seen that an experiment that uses a fluorescence image has the same result as shown in FIG. 8 and Table 1. In addition, from FIG .10, it can be seen that the nanoparticle complex has a much higher cellular uptake efficacy than the nanoparticle or the liposome-nanoparticle complex. Thus, it can be understood that the uptake efficacy is higher when the lipid structure is formed on the nanoparticle than when the liposome is simply bonded to the nanoparticle. Furthermore, it can be understood that the nanoparticle complex may be formed not only in the form of a bubble composed of lipids, but also in the form of a liposome or in the form of a sphere composed of lipids.
7. In addition, an adeno associate virus (AAV) is used as a vector that transports a therapeutic gene or a drug into a retinal tissue, and has a higher efficiency of transport than other vectors. However, the therapeutic gene or the drug is not transported up to a retinal pigment epithelium (RPE). From FIG. 11, it can be seen that the nanoparticle complex has a remarkably higher efficiency of cellular uptake than the nanoparticle. It can be understood that reduction of a specific reaction between a virus and a cellular receptor by the lipid structure increases a virus transfection ratio and thus increases a ratio of transport to a RPE layer. In addition, from FIG. 12, it can be seen that the nanoparticle complex in which the RNA nanoparticle coated with a polymer and hyaluronic acid is used has a remarkably higher cellular uptake efficacy than the nanoparticle. Furthermore, from FIGS. 13 and 14, it can be seen that the nanoparticle complexes that are manufactured using various types of lipids and various reactive groups have a much higher cellular uptake efficacy than the nanoparticle.

### <Implementation Example 8> Verification of Uptake of the Nanoparticle Complex by a Cell through Direct Penetration

### 1. Evaluation of the Efficiency of Cellular Uptake of the Nanoparticle Complex after Treatment with an Endocytosis Inhibitor

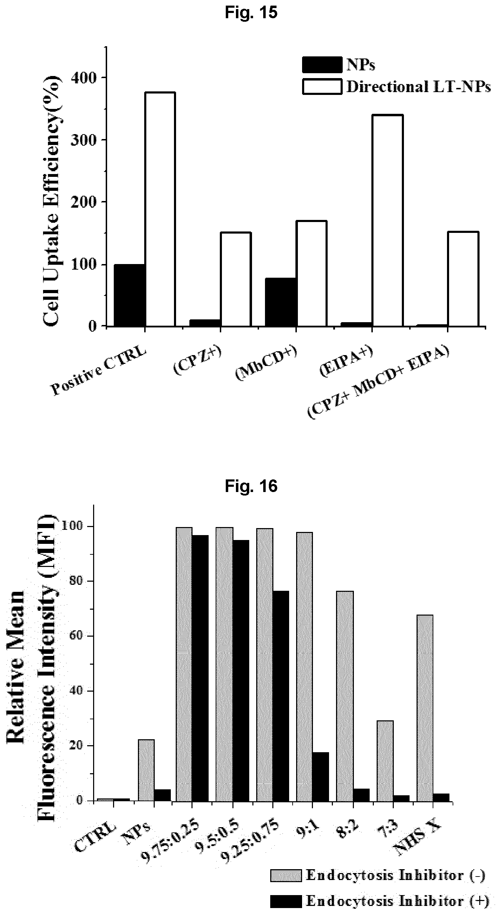

(1) Evaluation was made on the efficiency of uptake of each of the nanoparticles (NPs) formed in (1) of the first process in Implementation Example 1 and the nanoparticle complex (directional LT-NPs) formed in (2) of the fourth process in Implementation Example 1 by a cell treated with an endocytosis inhibitor. The nanoparticle having a size of 200 nm has been known to be ingested into the cell by a total of, largely, three mechanisms: macropinocytosis, clarthrin-independent endocytosis, and clarthrin-dependent endocytosis. Inhibitors that inhibit these mechanisms, respectively, were selected. First, A549 cells (1 × 10⁴) were treated with these inhibitors individually or at the same time for one hour. Then, A549 cells (1 × 10⁴) were treated for three hours with the nanoparticle and the nanoparticle complex that were marked with Alexa 488 fluorescent dye, and the result of the treatment was measured using the flow cytometry. The results of the measurement are normalized in terms of the nanoparticle and are shown in FIG. 15. As a macropinocytosis inhibitor, EIPA (5-(N-Ethyl-N-isopropyl) amiloride) was selected (at a concentration of 25 ug/ml), and a Na+/H+ exchange mechanism was inhibited. As a clathrin-dependent endocytosis inhibitor, CPZ (chlorpromazine) was selected (at a concentration of 20 ug/ml), and clathrin-coated pit formation was inhibited. As a clathrin-independent endocytosis inhibitor, MβCD (methyl-β-cyclodextrin) was selected (at a concentration of 3 mg/ml), and a cholesterol-dependent endocytic process was inhibited. In addition, as described above, A549 cells (1 × 10⁴) that were treated with the macropinocytosis inhibitor, the clathrin-dependent endocytosis inhibitor, and the clathrin-independent endocytosis inhibitor were treated with the nanoparticles (NPs) formed in the first process in Implementation Example 1 and the nanoparticle complex (directional LT-NPs) formed in (2) and (3) of the fourth process in Implementation Example 1 that were marked with Alexa 488 fluorescent dye. Then, fluorescence intensity was measured. The results of the measurement are shown in FIG. 16.
(2) From FIG. 15, it can be seen that the nanoparticles (NPs) have a greatly reduced cellular uptake efficacy due to CPZ and EIPA inhibitor. Furthermore, it can be seen that when three types of inhibitors were caused to act, and thus the endocytosis mechanisms were all inhibited, the cellular uptake of the nanoparticle seldom took place. Furthermore, it can be seen that cellular uptake efficacy of the nanoparticle complex (directional LT-NPs) is higher by 350% than that of the nanoparticle. It can be seen that when the endocytosis mechanisms were all inhibited with three types of inhibitors, the nanoparticle complex (directional LT-NPs) has an excellent cellular uptake efficacy. Thus, it can be understood that the nanoparticle complex directly penetrates not only endocytosis, but also a cell membrane. In addition, from FIG. 16, it can be seen that the cellular uptake efficacy of each of the nanoparticle complexes manufactured by mixing DSPC and DSPE-PEG-NHS2000 at ratios of 8:2 and 7:3 falls remarkably when three types of inhibitors are all caused to act. In contrast, it can be seen that the cellular uptake efficacy of the nanoparticle complex manufactured by mixing DSPC and DSPE-PEG-NHS2000 at or above a ratio of 9:1 has a much high cellular uptake efficacy although three inhibitors are all caused to act and thus endocytosis mechanisms are inhibited. Thus, it can be understood that in a case where a sufficient number of lipid structures having a sufficient length are formed on the nanoparticle, uptake of the nanoparticle occurs in the cell through direction penetration, such as translocation, instead of the endocytosis mechanism.

### 2. Evaluation of Cellular Uptake of the Nanoparticle Complex after HA Blocking

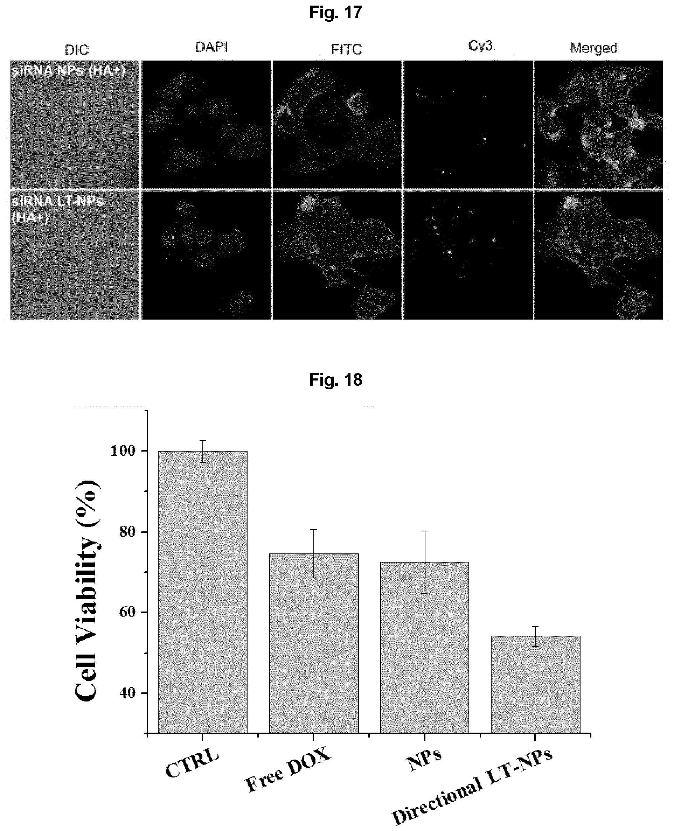

(1) Outermost portions of the nanoparticles (siRNA NPs) formed in the first process in Implementation Example 4 are coated with HA, and thus enter cells using CD44 receptors. Therefore, MCF-7 cells were excessively treated with HA, and the CD44 receptors were all blocked. Thereafter, MCF-7 cells were treated with the nanoparticle (siRNA NPs) formed in the first process in Implementation Example 4 and the nanoparticle complex (siRNA LT-NPs) formed in the second process in Implementation Example 4. Then, the result of the treatment was analyzed using the confocal microscope. The results of the analysis are shown in FIG. 17. The MCF-7 cells (1 × 10⁵) of which the nuclei were stained using DAPI and of which the cytoskeleton was stained using FITC were excessively treated with HA. Then, the nanoparticles and nanoparticle complex that were marked with Cy3 fluorescent dye were processed. Then, analysis was performed using uses the confocal microscope.
(2) From FIG. 17, it can be seen that because the cell is blocked with HA, the nanoparticle is ingested at a low efficiency of cellular uptake, but that the nanoparticle complex has a much high efficiency of cellular uptake although the CD44 receptor is blocked. Thus, it can be understood that the nanoparticle complex is ingested into a cell through direction penetration in addition to endocytosis or receptor binding.

### <Implementation Example 9> Evaluation of the Efficacy of the Nanoparticle Complex as an Anticancer Agent Transporter

1. Other conditions were set up to be the same as those in the first process in Implementation Example 1 and in (2) of the fourth process in Implementation Example 1, except that a solution that results from mixing doxorubicin with an albumin solution was added for reaction and that ethanol titration was then performed until the mixed solution became turbid. Then a doxorubicin-containing nanoparticle and a doxorubicin-containing nanoparticle complex were formed. Then, MCF-7/ADR, a cancer-resistant breast cancer cell line having resistance to anticancer, was seeded into well plates and was cultivated at a temperature of 37C° for six hours in a culture medium containing doxorubicin (100 mM, DOX), doxorubicin-containing nanoparticle (containing 100 mM of doxorubicin) and a doxorubicin-containing nanoparticle complex (containing 100 mM of doxorubicin) in each of the well plates. Then, the cancer-resistant breast cancer cell line was cultivated for 48 hours in a normal culture medium, and cell viability was performed. The results are shown in FIG. 18. For cell viability, an MTT assay and a trypan blue dye exclusion method are used. The cell viability is determined by cultivating a cell having 0.4% trypan blue dye and performing counting using a Neubauer hemocytometer. In the MTT assay, 96-well plates and 1.5mg/ml MTT reagent(3-(4,5-dimethylthiazol-2yl)-2,5-diphenyltetrazolium bromide) were used. Cultivation was performed for two hours with 15ul of MTT reagent, and then 200µl of DMSO was added to each well. The resulting culture plates was measured at 570 nm using a plate reader (manufactured by Bio Tek Instruments, Inc, Winooski, VT, USA).
2. From FIG. 18, it can be seen that when breast cancer cells are treated with doxorubicin of the same concentration, the use of the nanoparticle complex increases a cell death effect due to an anticancer agent not only in normal cancer cells, but also in cells with anticancer resistance to the anticancer agent.

### <Implementation Example 10> Evaluation of the Efficacy of the Nanoparticle Complex as a Dielectric Drug Transporter

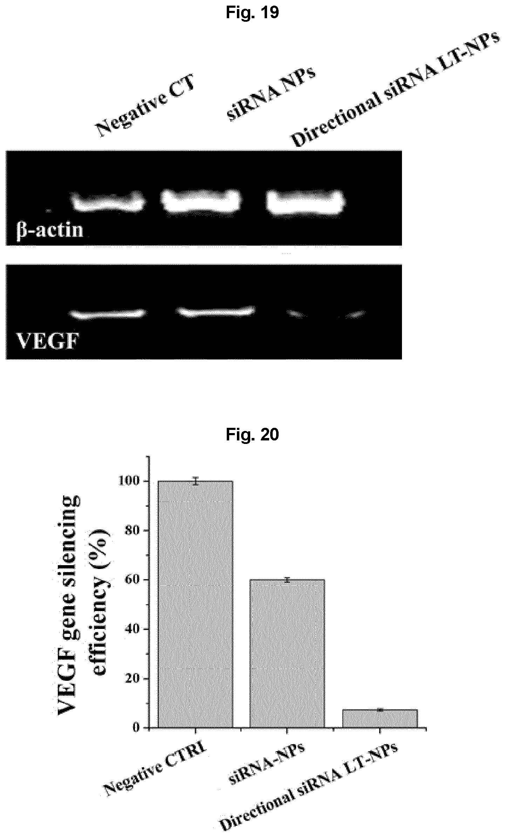

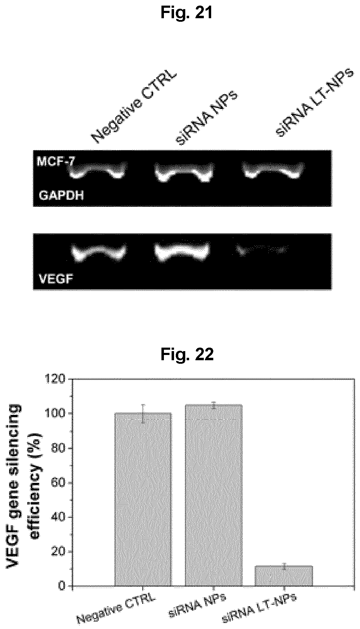

1. Gene silencing due to the nanoparticle (siRNA NPs) and the nanoparticle complex (directional siRNA LT-NPs) formed in the first and second processes, respectively, in Implementation Example 3 was verified, and the results of the verification are shown in FIGS. 19 and 20. In addition, gene silencing due to the nanoparticle (siRNA NPs) and the nanoparticle complex (directional siRNA LT-NPs) formed in the first and second processes, respectively, in Implementation Example 4 was verified, and the results of the verification are shown in FIGS. 21 and 22. MCF-7 cells (1 × 10⁵) were treated with each of the nanoparticle and the nanoparticle complex for three hours, and then cultivation was performed for 24 hours. Then, mRNA was extracted. PCR was performed on cells in the same way. In a PCR gel retardation assay, gene bands are stained with GelRed Nucleic Acid stain, and are visualized by a Gel Doc imaging device. FIGS. 20 and 22 illustrate values that are obtained by relatively quantifying the intensity of the gene band using an image analysis software Image-Pro.
2. From FIGS. 19 and 20, it can be seen that the nanoparticle complex, when used, has a much higher gene silencing efficiency than the nanoparticle composed of albumin and RNA. Thus, it can be understood that the nanoparticle complex can be effectively used as a dielectric drug transporter. In addition, from FIGS. 21 and 22, it can be seen that the RNA nanoparticle coated sequentially with a polymer and hyaluronic acid also has the same result as described above.

### <Implementation Example 11> Evaluation in Tumor Cell Spheroid

1. Unlike a normal adherent cancel cell, tumor cell spheroids is propagated using a 3D culture. Because a cancer cell grows in a floating state in a culture medium, the tumor cell spheroids grow in lumps. Because the result of the research shows that a shape of the cancel cells that grow in lumps mimics an extracellular matrix (ECM), an experiment was conducted in this model to verify in vitro whether or not tissue penetration according to lipid surface reforming occurs.
2. Formation of a Spheroid Cell

10 g of Poly (2-hydroxyethyl methacrylate) was added to 1 L of 100% pure ethanol 1L and was melted at a temperature of 60C°. Then, 3.3 ml of the melted Poly (2-hydroxyethyl methacrylate) was evenly dispersed on an entire plate on the 100 phi basis and was dried for 24 hours. Then, coating was performed, and MCF7 was seeded into the prepared plate. This state was maintained for 5 days. Thus, cells in which spheroids were formed were obtained.

### 3. Treatment of Spheroid Cell with the Nanoparticle Complex

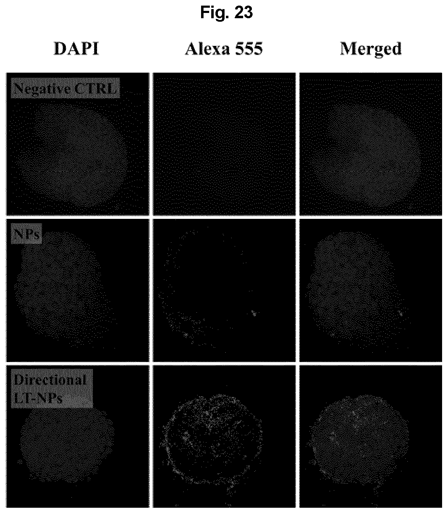

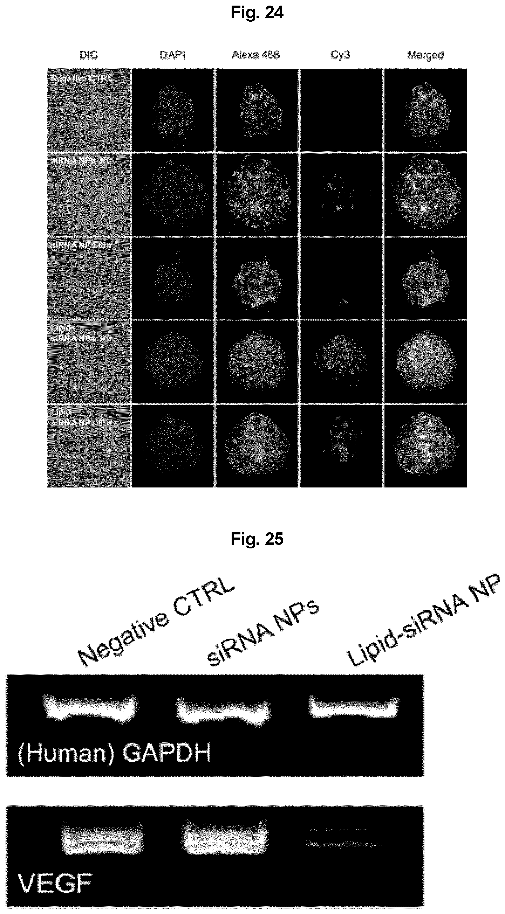

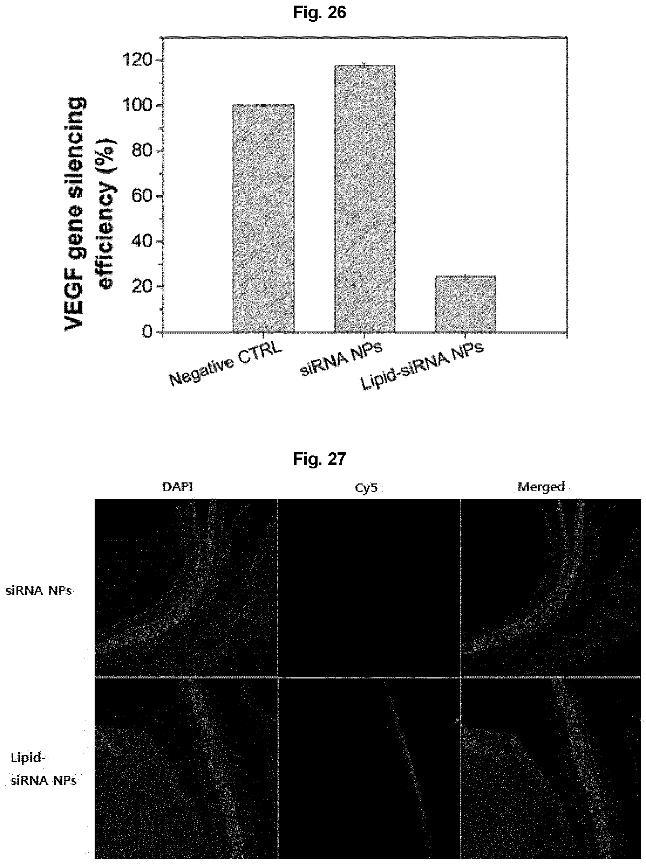

(1) Imaging was performed in order to evaluate the efficiency of uptake of each of the nanoparticles (NPs) formed in the first process in Implementation Example 1 and the nanoparticle complex (directional LT-NPs) formed in (2) of the fourth process in Implementation Example 1 by the spheroid cell. The results of the imaging are shown in FIG. 23. MCF-7 cells (1 × 10⁵) in the form of a spheroid that were formed in the second process in Implementation Example 11, nuclei of MCF-7 cells being stained using DAPI, were treated with the nanoparticle and the nanoparticle complex that were marked with Alexa 555 fluorescent dye. At a point in time when three hours elapsed, the results was measured using the confocal microscope.
(2) In addition, imaging was performed in order to evaluate the efficiency of uptake of each of the nanoparticles (siRNA NPs) formed in the first process in Implementation Example 4 and the nanoparticle complex (lipid siRNA NPs) formed in the second process in Implementation Example 4 by the spheroid cell. The results of the imaging are shown in FIG. 24. MCF-7 cells (1 × 10⁵) in the form of a spheroid that were formed in the second process according to Implementation Example, the nuclei of MCF-7 cells being stained using DAPI and the cytoskeletons thereof being stained using Alexa 488, were treated with the nanoparticle and the nanoparticle complex that were marked with Cy3 fluorescent dye. At a point in time when three hours and six hours elapsed, the result was measured using the confocal microscope.
(3) In addition, spheroid cell gene silencing due to the nanoparticles (siRNA NPs) formed in the first process in Implementation Example 4 and the nanoparticle complex (lipid-siRNA NP) formed in the second process in Implementation Example 4 was verified. The results are shown in FIGS. 25 and 26. For the gene silencing, MCF-7 cells (1 × 10⁵) in the form of a spheroid were treated with each of the nanoparticle and the nanoparticle complex for 3 hours, and then cultivation was performed for 24 hours. Then, mRNA was extracted. PCR was performed on cells in the same way. In the PCR gel retardation assay, gene bands were stained with GelRed Nucleic Acid stain, and are visualized by the Gel Doc imaging device. FIGS. 26 illustrates values that were obtained by relatively quantifying the intensity of the gene band using the image analysis software Image-Pro.
(4) From FIGS. 23 to 26, it can be seen that the nanoparticle complex has a much higher efficiency of cellular uptake than the nanoparticle. Accordingly, it can be understood that the nanoparticle complex has an excellent cellular uptake not only in a simple in-vitro environment, but also in an environment that mimics the in-vitro environment. Thus, it can be understood that the nanoparticle complex has an excellent efficiency of penetration into a tissue.

### <Implementation Example 12> Evaluation of Cellular Uptake of the Nanoparticle Complex in an Animal Model

1. Imaging was performed in order to evaluate the efficiency of uptake of each of the nanoparticles (siRNA NPs) formed in the first process in Implementation Example 4 and the nanoparticle complex (lipid siRNA NPs) formed in the second process in Implementation Example 4 by a dog cell. The results of the imaging are shown in FIG. 27. In the animal model experiment, a 5 ul eye drop that contains each of the nanoparticle and the nanoparticle that are marked with Cy5 fluorescent dye was prepared, and the eye drop was intravitreally injected into an eyeball of a Brown Norway Rat. 30 days after the eye drop was injected, the eyeball was extracted, and was fixed with 4% PFA. Then, the eyeball was immersed in 20% sucrose for 30 minutes. Then, the eyeball was taken out of the sucrose, and was mixed with O.C.T compound. The O.C.T compound was poured into Cryomold and was frozen. Then, an eyeball tissue was removed from the Cryomold, was sliced with a cryosection machine, and was spread out in a manner that is not wrinkled. A DAPI solution was caused to react for five minutes, and thus a nucleus of the tissue was stained. Then, the result was measured using the confocal microscope.
2. From FIG. 27, it can be seen that the nanoparticle complex remains in the eyeball tissue for a longer time than the nanoparticle. Thus, it can be understood that the nanoparticle complexes has an excellent efficiency of cellular uptake.

Various embodiments of the present disclosure are described above, and the various embodiments are only examples in which the technical ideal of the present disclosure is embodied. Any modification or alteration example should be interpreted as falling within the scope of the present disclosure, as long as it embodies the technical idea of the present disclosure.

## Claims

1. A nanoparticle complex comprising:
- a nanoparticle that is ingestible into a cell; and
- a lipid-based lipid structure bonded to one portion of an outer surface of the
nanoparticle and improving a cellular uptake efficiency of the nanoparticle, wherein the nanoparticle comprises a first reactive group, the lipid structure comprises a second reactive group chemically bonded to the first reactive group of the nanoparticle, the first reactive group and the second reactive group are chemically bonded to each other, and the lipid structure is thus bonded to the nanoparticle.

2. The nanoparticle complex of claim 1, wherein the lipid structure is formed by recombining lipidomes each being composed of a mixture of a lipid to which the second reactive group is bonded and a lipid to which the second reactive group is not bonded, and generation of the lipid structure bonded to the nanoparticle and formation of a shape thereof are controlled by adjusting a mixture ratio between the lipid to which the second reactive group is bonded and the lipid to which the second reactive group is not bonded.

3. The nanoparticle complex of claim 2, wherein the lipid to which the second reactive group is bonded and the lipid to which the second reactive group is not bonded are used at a molar ratio of 1:2.33 to 99.

4. The nanoparticle complex of claim 1, wherein a lipid head of the lipid structure that has hydrophilicity is positioned on an outer lateral surface of the lipid structure, a lipid tail of the lipid structure that has hydrophilicity is positioned inside the lipid structure, and the lipid structure has the form of a long tube when viewed as a whole.

5. The nanoparticle complex of claim 1, wherein the nanoparticle has a diameter of 50 to 500 nm, and the lipid structure has a length of 50 to 300 nm and has a width of 3 to 20 nm.

6. The nanoparticle complex of claim 1, wherein the nanoparticle complex is ingested into the cell by endocytosis and by directly penetrating a cell membrane.

7. The nanoparticle complex of claim 1, wherein the lipid structure is capable of improving the efficiency of uptake of the nanoparticle by a spheroid cell.

8. The nanoparticle complex of claim 1, wherein the nanoparticle includes an anticancer agent and is capable of improving the efficiency of death of a tumor cell having resistance to anticancer.

9. The nanoparticle complex of claim 1, wherein the nanoparticle includes a dielectric or is composed of the dielectric, the nanoparticle complex being used for gene therapy.

10. A method of manufacturing a nanoparticle, the method comprising:
- forming a nanoparticle having a first reactive group;
- forming a micro-based lipid-based lipidome containing a second reactive group chemically bonded to the first reactive group;
- forming a lipidome-nanoparticle complex by mixing the nanoparticle and the lipidome, bonding the first reactive group and the second reactive group to each other, and thus bonding the nanoparticle to an outer surface of the lipidome; and
- breaking the lipidome by applying a mechanical force to the lipidome-nanoparticle complex formed and thus forming a lipid structure bonded to one portion of an outer surface of the nanoparticle,
wherein in the forming of the lipidome, the lipidome is formed using a mixture of a lipid to which the second reactive group is bonded and a lipid to which the second reactive group is not bonded, the nanoparticle is ingested into a cell, and the lipid structure improves the efficiency of cellular uptake of the nanoparticle.

11. The method of claim 10, wherein generation of the lipid structure bonded to the nanoparticle and formation of a shape thereof are controlled by adjusting a mixture ratio between the lipid to which the second reactive group is bonded and the lipid to which the second reactive group is not bonded.

12. The method of claim 10, wherein in the breaking of the lipidome, in a case where a mechanical force is applied to the lipidome-nanoparticle complex and after a predetermined time elapses, the lipidome is broken, phospholipids that constitute the lipidome are recombined, and thus the lipid structure in the form of a tube that is bonded to the nanoparticle is formed.
